(19) Europäisches Patentamt
European Patent Office
Office européen des brevets

(11) **EP 3 027 298 B1**

(12) **EUROPÄISCHE PATENTSCHRIFT**

(45) Veröffentlichungstag und Bekanntmachung des Hinweises auf die Patenterteilung:
**15.05.2024 Patentblatt 2024/20**

(21) Anmeldenummer: **14744819.5**

(22) Anmeldetag: **25.07.2014**

(51) Internationale Patentklassifikation (IPC):
**B01D 61/02** (2006.01)    **B01D 61/12** (2006.01)
**C07C 45/50** (2006.01)

(52) Gemeinsame Patentklassifikation (CPC):
(C-Sets verfügbar)
**B01D 61/12; B01D 61/026;** B01D 2311/04;
B01D 2311/08; B01D 2311/106; B01D 2311/25;
B01D 2317/025; B01D 2317/027        (Forts.)

(86) Internationale Anmeldenummer:
**PCT/EP2014/066028**

(87) Internationale Veröffentlichungsnummer:
**WO 2015/014741 (05.02.2015 Gazette 2015/05)**

(54) **MEMBRANKASKADE MIT SINKENDER TRENNTEMPERATUR**

MEMBRANE CASCADE WITH FALLING SEPARATING TEMPERATURE

CASCADE DE MEMBRANES À TEMPÉRATURE DE SÉPARATION DESCENDANTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.07.2013 DE 102013215004**

(43) Veröffentlichungstag der Anmeldung:
**08.06.2016 Patentblatt 2016/23**

(73) Patentinhaber: **Evonik Oxeno GmbH & Co. KG 45772 Marl (DE)**

(72) Erfinder:
• **PRISKE, Markus**
  **Mobile, AL, Alabama 36609 (US)**
• **FRANKE, Robert**
  **45772 Marl (DE)**
• **HAMERS, Bart**
  **NL-5961 VG Horst (NL)**
• **SCHMIDT, Patrick**
  **68165 Mannheim (DE)**
• **GÓRAK, Andrzej**
  **58453 Witten (DE)**

(74) Vertreter: **Evonik Patent Association c/o Evonik Industries AG IP Management Bau 1042A/PB 15 Paul-Baumann-Straße 1 45772 Marl (DE)**

(56) Entgegenhaltungen:
EP-A1- 0 521 784      EP-A1- 0 700 711
EP-A1- 1 931 472      EP-A1- 2 226 113
EP-A2- 0 374 615      EP-A2- 0 823 282
CN-B- 101 591 254    DE-A1-102009 001 225

• LIN ET AL: "Nanofiltration membrane cascade for continuous solvent exchange", CHEMICAL ENGINEERING SCIENCE, OXFORD, GB, Bd. 62, Nr. 10, 22. April 2007 (2007-04-22), Seiten 2728-2736, XP022042696, ISSN: 0009-2509, DOI: 10.1016/J.CES.2006.08.004
• WEIMING EUGENE SIEW ET AL: "Molecular separation with an organic solvent nanofiltration cascade - augmenting membrane selectivity with process engineering", CHEMICAL ENGINEERING SCIENCE, Bd. 90, 1. März 2013 (2013-03-01), Seiten 299-310, XP055141663, ISSN: 0009-2509, DOI: 10.1016/j.ces.2012.10.028

(52) Gemeinsame Patentklassifikation (CPC): (Forts.)

C-Sets
B01D 2311/04, B01D 2311/263;
B01D 2311/08, B01D 2311/25, B01D 2311/04

**Beschreibung**

Die Erfindung betrifft ein Verfahren zur Trennung eines Stoffgemisches mit Hilfe einer mindestens zwei Stufen aufweisenden Membrankaskade, bei welchem in jeder Stufe eine Stofftrennung an mindestens einer Membran bei einer der jeweiligen Stufe zugeordneten Trenntemperatur erfolgt.

Der Verfahrenstechniker versteht unter einer Trennoperation eine Maßnahme zur qualitativen Trennung von Stoffgemischen, bei der ein Eingangs-Stoffgemisch enthaltend mehrere Komponenten in mindestens zwei Ausgangs-Stoffgemische überführt wird, wobei die erhaltenen Ausgangs-Stoffgemische eine andere mengenmäßige Zusammensetzung als das Eingangs-Stoffgemisch aufweisen. Die erhaltenen Ausgangs-Stoffgemische weisen in der Regel eine besonders hohe Konzentration der gewünschten Komponente auf, bestenfalls handelt es sich um Reinprodukte. Der Aufreinigungsgrad bzw. die Trennschärfe steht meist im Zielkonflikt mit der Durchsatzleistung und dem erforderlichen apparativen Aufwand und dem Energieeinsatz.

In so genannten Membrantrennverfahren wird das aufzutrennende Stoffgemisch 0 als Feed F auf eine Membran 1 gegeben, welche für die im Stoffgemisch 0 enthaltenen Komponenten eine unterschiedliche Durchlässigkeit aufweist. Komponenten, welche die Membran 1 besonders gut durchschreiten, werden als Permeat P jenseits der Membran 1 gesammelt und abgeführt. Komponenten, welche die Membran 1 bevorzugt zurückhält, werden diesseits der Membran 1 als Retentat R gesammelt und abgeführt.

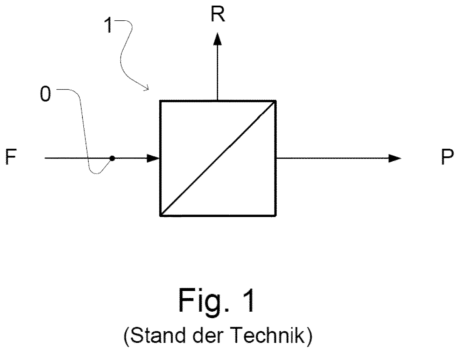

Die Lage der Ströme an einer Membran sind aus Figur 1 ersichtlich.

Figur 1: einfaches Membranverfahren (Stand der Technik)

In der Membrantechnik kommen unterschiedliche Trenneffekte zu tragen. Insbesondere werden nicht nur Größenunterschiede der Komponenten (mechanischer Siebeffekt), sondern auch Lösungs- und Diffusionseffekte genutzt. Je dichter die trennaktive Schicht der Membran wird, desto dominierender werden Lösungs- und Diffusionseffekte. Das Abtrennungsverhalten einer Membran ist mithin mit dem eines einfachen Filters, der sich lediglich des Siebeffekts bedient, nicht vollumfänglich vergleichbar.

Eine hervorragende Einführung in die Membrantechnologie bietet:

Melin / Rautenbach: Membranverfahren. Grundlagen der Modul- und Anlagenauslegung. Springer, Berlin Heidelberg 2004.

Ein Vorteil der Membranverfahren gegenüber anderen Trennverfahren ist der geringe Energiebedarf. So ist in der Regel nur mechanische Leistung erforderlich, um Pumpen zur Aufrechterhaltung der erforderlichen Ströme und Drücke zu betreiben. Anders als etwa thermische Trennverfahren benötigen Membrantrennverfahren keine Wärmeenergie.

Ein spezifischer Nachteil von Membrantrennverfahren ist, dass diese noch vergleichsweise junge Technologie mit der Verfügbarkeit der Membranen steht und fällt. So erfordert die effektive Abtrennung spezieller Komponenten aus dem Stoffgemisch oft ein spezielles Membranmaterial, welches nicht immer in ausreichend großen Mengen verfügbar oder gar nicht bekannt ist. Die Auftrennung großvolumiger Stoffströme erfordert aber sehr große Membranflächen und damit verbunden entsprechend viel Material und hohe Investitionskosten.

Dies gilt umso mehr, wenn ein besonders großer Rückhalt einer bestimmten Komponente gefordert ist: Der Rückhalt ist ein Maß für die Fähigkeit einer Membran, eine im Feed enthaltene Komponente im Retentat anzureichern bzw. im Permeat abzureichern. Der Rückhalt R berechnet sich aus dem permeatseitigen Stoffmengenanteil der betrachteten Komponente an der Membran $x_P$ und dem retentatseitigen Stoffmengenanteil der betrachteten Komponente an der Membran $x_R$ wie folgt:

$$R = 1 - x_P / x_R$$

Die Konzentrationen $x_P$ und $x_R$ sind dabei direkt an den beiden Membranseiten zu messen.

Wenn die Membran hinsichtlich der zurückzuhaltenden Komponente einen geringen Rückhalt aufweist - etwa weil kein effektiveres Membranmaterial bekannt ist - muss der zu trennende Stoffstrom mehrfach auf die Membran gegeben werden, um insgesamt einen ausreichenden Rückhalt zu erzielen. Dies geschieht entweder durch Rückführung des Permeats in den Feed derselben Membran oder durch Beaufschlagen einer zweiten Membran mit dem Permeat der ersten Membran.

Ein Membrantrennverfahren, bei welchen der aufzutrennende Stoffstrom durch mehrere Membranen permeiert, wird mehrstufig genannt. Mehrstufige Membrantrennverfahren werden in sogenannten Membrankaskaden durchgeführt; das sind Anordnungen von mehreren einzelnen Membranen, die seriell und/oder parallel miteinander verschaltet sind.

Membrankaskaden umfassen in der Regel auch Pumpen zur Aufrechterhaltung des als Triebkraft über die Membran erforderlichen Transmembrandruckes sowie etwaige Rückführleitungen, welche Teilströme innerhalb der Kaskade mehrfach über einzelne Stufen leiten.

Wenngleich Membrankaskaden sehr komplex aufgebaut sein können, lässt sich doch jede Membrankaskade als Blackbox wie eine einzelne Membran auffassen, welche über die in Figur 1 dargestellten Grundanschlüsse Feed, Retentat und Permeat verfügt. Um die Ströme der Blackbox von den internen Strömen der Membrankaskade zu unterscheiden, wird fortan von einem resultieren Permeat bzw. resultierenden Retentat gesprochen, wenn von dem Retentat bzw. Permeat einer gesamten Membrankaskade die Rede ist.

Wie bereits erwähnt, können innerhalb einer Membrankaskade mehrere Membranen parallel und/oder seriell verschaltet sein. Dies geschieht in der Technik recht häufig, da Membranen technisch in der Regel als Membranmodul mit einer begrenzten Membranfläche ausgeführt sind und die erforderliche Gesamtfläche mittels mehrerer parallel geschalteter Membranmodule bereitgestellt wird.

Für die Charakterisierung einer Membrankaskade ist mithin nicht die Anzahl von Membranen innerhalb einer Membrantrenneinrichtung interessant, sondern vielmehr die Anzahl der Stufen. Unter einer Stufe versteht der Membrantechniker einen Trennschritt, bei welchem an einer oder mehreren Membranen ein Druckabfall eintritt. Dieser Druckabfall ist die Differenz zwischen Feed und Permeat an der betrachteten Membran und wird Transmembrandruck genannt. Ohne diesen Druckabfall findet keine Trennung an der Membran statt. Da bei einem mehrstufigen Membranprozess der nach der ersten Stufe eingetretene Druckverlust wieder aufgebaut werden muss, lässt sich die Anzahl der Stufen auch an der Anzahl der Pumpen zur Einstellung bzw. Wiederherstellung des Transmembrandruckes ablesen. Alternativ, jedoch selten in der Praxis umgesetzt, kann die transmembrane Druckdifferenz von zwei oder mehr Stufen auch durch eine Pumpe erzeugt. Hierzu erzeugt die Pumpe vor einer Stufe in Summe den Transmembrandruck von zwei oder mehr Stufen. D.h. der Permeatdruck der vorgelagerten Stufe bildet den Feeddruck der nachgelagerten Stufe.

Es sind zwei grundlegende Verschaltungsformen von Membrankaskaden bekannt, nämlich die Verstärkerkaskade (engl.: enriching cascade) und die Abtriebskaskade (engl.: stripping cascade). Darüber hinaus existieren Mischformen, die sich aber auf die beiden Basisverschaltungen zurückführen lassen.

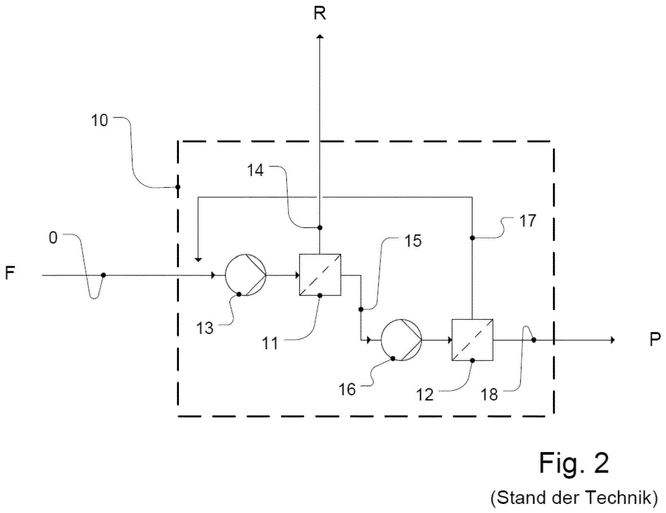

Den Aufbau einer Verstärkerkaskade mit zwei Membrantrennstufen zeigt Figur 2.

Figur 2: zweistufige Verstärkerkaskade (Stand der Technik)

Die Verstärkerkaskade 10 kann als Blackbox aufgefasst werden, die wie eine einzelne Membran das aufzutrennende Stoffgemisch 0 als Feed F entgegennimmt und es in ein resultierendes Retentat R und ein resultierendes Permeat P auftrennt. Die Verstärkerkaskade 10 umfasst zwei in Richtung des Permeatflusses hintereinander geschaltete Stufen 11, 12 die ihrerseits eine oder mehrere Membranen umfassen. Der anströmende Feed F wird von einer Förderpumpe 13 auf die erste Stufe 11 gefördert. Dort findet eine erste Stofftrennung an einer Membran statt, so dass ein Retentat der ersten Stufe 14 und ein Permeat der ersten Stufe 15 anfällt. Das Retentat der ersten Stufe 14 entspricht dem resultierenden Retentat R der Verstärkerkaskade 10 und wird aus dem Trennprozess ausgeschleust.

Das Permeat 15 der ersten Stufe 11 hingegen wird als Feed der zweiten Stufe 12 aufgegeben. Um den an der ersten Stufe 11 eingetretenen Druckverlust (Transmembrandruck) auszugleichen, ist eine Hochdruckpumpe 16 zwischen der ersten und zweiten Stufe vorgesehen.

An der zweiten Stufe 12 findet erneut eine Stofftrennung an mindestens einer Membran statt, so dass ein Retentat 17 der zweiten Stufe und ein Permeat 18 der zweiten Stufe anfällt. Das Permeat 18 der zweiten Stufe hat mithin beide Stufen 11, 12 überwunden und weist deswegen eine besonders hohe Reinheit auf. Es wird als resultierendes Permeat P aus dem Prozess ausgeschleust.

Das Retentat 17 der zweiten Stufe 12 indes wird in den Feed der ersten Stufe 11 rezykliert, um erneut aufgereinigt zu werden.

Da das resultierende Permeat P eine Verstärkungskaskade 10 alle Stufen 11, 12 permeiert hat, dienen Verstärkungskaskaden dazu, eine im anströmenden Stoffstrom 0 enthaltene, von den Membranen bevorzugt durchgelassene Komponente im resultierenden Permeat P anzureichern.

Gilt es hingegen, eine bevorzugt von der eingesetzten Membran zurückgehaltene Komponente im Retentat anzureichern, wird eine Abtriebskaskade eingesetzt.

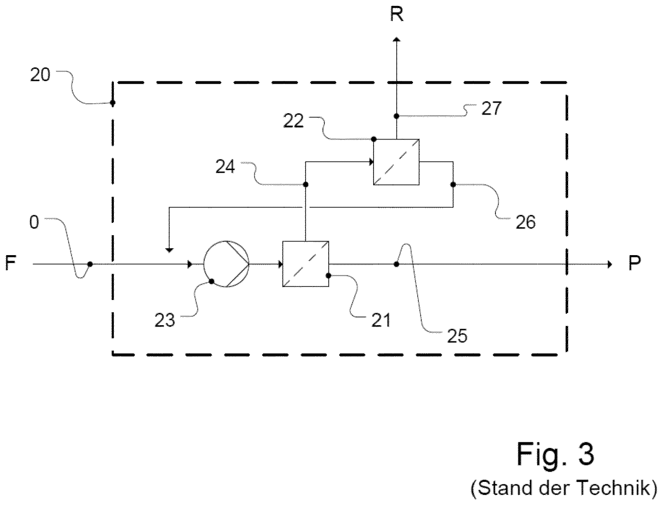

Den Aufbau einer Abtriebskaskade mit zwei Membrantrennstufen zeigt Figur 3:

Figur 3: zweistufige Abtriebskaskade (Stand der Technik)

Die Abtriebskaskade 20 ist wiederum als Blackbox entsprechend einer einzelnen Membran aufzufassen, welche mit dem anströmenden Stoffstrom 0 als Feed F beaufschlagt wird welche den Feed F in ein resultierendes Retentat R und ein resultierendes Permeat P auftrennt.

Die Abtriebskaskade 20 unterscheidet sich von der in Figur 2 dargestellten Verstärkerkaskade 10 dadurch, dass die Stufen in Richtung Retentatflusses hintereinander angeordnet sind. Zur Stofftrennung dienen bei der in Figur 3 gezeigten zweistufigen Abtriebskaskade zwei Stufen 21, 22.

Zwecks Trennung des Stoffstroms 0 wird dieser der ersten Stufe 21 der Abtriebskaskade 20 aufgegeben und dort in Permeat 24 und Retentat 25 getrennt. Zur Erzeugung des notwenigen Transmembrandruckes ist eine Druckerhöhungspumpe 23 vorgesehen. Das Permeat 25 der ersten Stufe 21 wird aus der Abtriebskaskade 20 ausgeschleust und entspricht somit dem resultierenden Permeat P.

Das Retentat 24 der ersten Stufe 21 wird indes als Feed der zweiten Stufe 22 aufgegeben. Hierzu ist keine zwischen der ersten Stufe 21 und zweiten Stufe 22 angeordnete Pumpe erforderlich, da der Transmembrandruck stets in Richtung des Permeats abfällt, sodass der Druck des Retentats 24 - abgesehen von Strömungsverlusten - im Wesentlichen dem des Feeds der ersten Stufe 21 entspricht.

Innerhalb der zweiten Stufe 22 findet abermals eine Stofftrennung an mindestens einer Membran statt. Das dabei anfallende Retentat der zweiten Stufe 27 wird als resultierendes Retentat R aus der Abtriebskaskade 20 ausgeschleust. Da es keine Membran der beiden Stufen 21, 22 überwunden hat, ist es besonders reich an durch die Membranen bevorzugt zurückgehaltenen Komponenten.

Das Permeat 26 der zweiten Stufe 22 wird indes rezykliert und dem Feed der ersten Stufe 21 beigemischt. Die Druckerhöhungspumpe 23 gleicht den in der zweiten Stufe 22 erlittenen Druckverlust des Permeats 26 wieder aus.

Auf dem Gebiet der Wasserentsalzung, der Gastrennung und der pharmazeutischen Stoffreinigung sind Membrankaskaden bereits weit verbreitet.

Ein vergleichbar junges Einsatzgebiet von Membrankaskaden hingegen ist die Katalysatorabtrennung aus homogen katalysierten chemischen Reaktionen.

Soweit hier von einer katalytischen Reaktion die Rede ist, ist eine chemische Reaktion gemeint, bei der mindestens ein Edukt in Gegenwart eines Katalysators in mindestens ein Produkt umgesetzt wird. Edukt und Produkt werden gemeinschaftlich als Reaktionsteilnehmer bezeichnet. Der Katalysator wird während der Reaktion im Wesentlichen nicht verbraucht; abgesehen von üblichen Alterungs- und Zersetzungserscheinungen.

Die Reaktion wird in einer örtlich begrenzten Reaktionszone durchgeführt. Dies ist einfachstenfalls ein Reaktor jedweder Bauart, es kann sich aber auch um eine Mehrzahl von miteinander verschalteten Reaktoren handeln.

Das aus der Reaktionszone kontinuierlich oder diskontinuierlich entnommene Material wird hier als Reaktionsgemisch bezeichnet. Das Reaktionsgemisch umfasst zumindest das Zielprodukt der Reaktion. Je nach technischer Reaktionsführung kann es auch nicht umgesetzte Edukte, mehr oder weniger gewünschte Folge- oder Begleitprodukte aus Folge- bzw. Nebenreaktionen und Lösemittel enthalten. Darüber hinaus kann das Reaktionsgemisch auch den Katalysator umfassen.

Katalytisch durchgeführte, chemische Reaktionen lassen sich hinsichtlich des Phasenzustandes des eingesetzten Katalysators in zwei Gruppen unterteilen: Zum einen sind hier die heterogen katalysierten Reaktionen zu nennen, bei denen der Katalysator als Feststoff in der Reaktionszone vorliegt und von Reaktionsteilnehmern umgeben ist. Bei der homogenen Katalyse ist der Katalysator hingegen im Reaktionsgemisch aufgelöst. Homogen gelöste Katalysatoren sind katalytisch meist deutlich effektiver als heterogene Katalysatoren.

Bei jeder katalytisch durchgeführten Reaktion ist es erforderlich, den Katalysator aus dem Reaktionsgemisch abzutrennen. Der Grund dafür ist, dass der Katalysator während der Reaktion praktisch nicht verbraucht wird und daher wiederverwendet werden kann. Außerdem ist der Katalysator meist deutlich wertvoller als das damit hergestellte Produkt. Katalysatorverlust ist daher tunlichst zu vermeiden.

Die Katalysatorabtrennung lässt sich bei heterogen katalysierten Reaktionen technisch einfach bewerkstelligen: Der feste Katalysator verbleibt einfach in der Reaktionszone währenddessen das flüssige und/oder gasförmige Reaktionsgemisch aus dem Reaktor abgezogen wird. Die Abtrennung des Heterogenkatalysators aus dem Reaktionsgemisch erfolgt damit mechanisch und unmittelbar innerhalb der Reaktionszone.

Die Abtrennung eines Homogenkatalysators aus einem Reaktionsgemisch ist indes deutlich anspruchsvoller, da der Homogenkatalysator im Reaktionsgemisch gelöst ist. Eine simple mechanische Abtrennung scheidet damit aus. Deswegen wird bei homogenkatalytischen Prozessen der Katalysator im Reaktionsgemisch gelöst aus der Reaktionszone entnommen und in einem gesonderten Schritt von dem Reaktionsgemisch abgetrennt. Die Katalysatorabtrennung erfolgt in der Regel außerhalb der Reaktionszone. Der abgetrennte Katalysator wird in die Reaktionszone zurückgeführt.

Besondere technische Probleme treten bei der Abtrennung von Katalysatorsystemen auf, die empfindlich auf Zustandsänderungen reagieren und deswegen besonders schonend abgetrennt werden müssen.

So werden einige Reaktionen in Gegenwart von hocheffektiven, aber auch hochempfindlichen Homogenkatalysatorsystemen wie zum Beispiel metallorganischen Komplexverbindungen durchgeführt. Das im Katalysatorsystem enthaltene Metall lässt sich nahezu vollständig abtrennen und in der Anlage zurück halten. Allerdings wird die Komplexverbindung bei unsachgemäßer Abtrennung leicht zerstört, sodass der zurückgehaltene Katalysator inaktiv und damit unbrauchbar wird.

Die Abtrennung von homogen gelösten Katalysatorsystemen aus Reaktionsmischungen bei möglichst geringen Material- und Aktivitätsverlust stellt mithin eine anspruchsvolle verfahrenstechnische Aufgabe dar.

Diese Aufgabe stellt sich insbesondere auf dem Gebiet der rhodiumkatalysierten Hydroformylierung.

Die Hydroformylierung - auch Oxo-Reaktion genannt - ermöglicht es, Olefine (Alkene) mit Synthesegas (Gemisch aus Kohlenmonoxid und Wasserstoff) in Aldehyde umzusetzen. Die erhaltenen Aldehyde weisen dann entsprechend ein Kohlenstoff-Atom mehr auf als die eingesetzten Olefine. Durch anschließende Hydrierung der Aldehyde entstehen Alkohole, die aufgrund ihrer Genese auch "Oxo-Alkohole" genannt werden.

Grundsätzlich sind alle ethylenisch ungesättigte Verbindungen der Hydroformylierung zugänglich, in der Praxis werden jedoch meist solche Olefine als Substrat in der Hydroformylierung eingesetzt, die zwei bis 20 Kohlenstoffatome aufweisen. Da die durch Hydroformylierung und Hydrierung erhältlichen Alkohole vielfältig eingesetzt werden können - etwa als Weichmacher für PVC, als Detergentien in Waschmitteln und als Riechstoffe - wird die Hydroformylierung großindustriell praktiziert.

Wichtige Kriterien zur Unterscheidung von technischen Hydroformylierungsverfahren sind neben dem eingesetzten Substrat das Katalysatorsystem, die Phasenaufteilung im Reaktor und die Technik zum Austrag der Reaktionsprodukte aus dem Reaktor. Ein weiterer technisch relevanter Aspekt ist die Anzahl der durchgeführten Reaktionsstufen.

Industriell kommen entweder Cobalt- oder Rhodium-basierte Katalysatorsysteme zum Einsatz, wobei letztere mit einer Organophosphorverbindung als Ligand komplexiert werden. In der Regel kommen dabei Phosphin-, Phosphit- oder Phosphoramidit-Verbindungen als Ligand zum Einsatz. Diese Katalysatorsysteme liegen allesamt als Homogenkatalysator gelöst in der Reaktionsmischung vor.

Die Hydroformylierungsreaktion wird meist zweiphasig durchgeführt, mit einer flüssigen Phase, welche die Olefine, den gelösten Katalysator und die Produkte enthält sowie einer Gasphase, welche im Wesentlichen von Synthesegas gebildet wird. Die Wertprodukte werden dann entweder flüssig aus dem Reaktor abgezogen ("liquid recycle") oder gasförmig mit dem Synthesegas ausgetragen ("gas recycle"). Ein Sonderfall stellt das Ruhrchemie/Rhône-Poulenc-Verfahren dar, bei dem der Katalysator sich in einer wässrigen Phase befindet.

Einige Hydroformylierungsverfahren werden auch in Gegenwart eines Lösungsmittels durchgeführt. Dabei handelt es sich beispielsweise um Alkane, die in dem Einsatzgemisch enthalten sind.

Da sich die Erfindung im Wesentlichen mit Stofftrennung, insbesondere mit der Abtrennung des Katalysatorsystems aus einem Reaktionsgemisch befasst, welches aus einer homogen katalysierten Reaktion wie insbesondere einer Hydroformylierung stammt, wird hinsichtlich der Chemie und Reaktionstechnik der Hydroformylierung auf den umfangreichen Stand der Technik verwiesen. Besonders lesenswert sind:

Falbe, Jürgen: New Syntheses with Carbon Monoxide. Springer, 1980. (Standard-Werk zur Hydroformylierung)

Pruett, Roy L.: Hydroformylation. Advances in Organometallic Chemistry. Vol. 17 Seiten 1 bis 60, 1979 (Übersichtsartikel)

Frohning, Carl D. und Kohlpaintner, Christian W.: Hydroformylation (Oxo Synthesis, Roelen Reaction). Applied homogeneous catalysis with organometallic compounds. Wiley, 1996. Seiten 29 bis 104. (Übersichtsartikel)

Van Leeuwen, Piet W.N.M und Claver, Carmen (Edit.): Rhodium Catalyzed Hydroformylation. Catalysis by Metal Complexes. Volume 22. Kluwer, 2000.

(Monographie zur Rh-katalysierten Hydroformylierung. Schwerpunkt auf der Chemie, aber auch verfahrenstechnische Aspekte werden diskutiert.)

R. Franke, D. Selent und A. Börner: "Applied Hydroformylation", Chem. Rev., 2012, DOI:10.1021/cr3001803 (Übersicht des aktuellen Forschungsstands).

Ein wesentlicher Schlüssel zu einer erfolgreichen, großindustriellen Durchführung von Rhbasierten, homogen katalysierten Hydroformylierungen ist die Beherrschung der Katalysatorabtrennung.

Dies liegt zum einen daran, dass Rh ein sehr teures Edelmetall ist, dessen Verlust es tunlichst zu vermeiden gilt. Aus diesem Grunde muss das Rhodium aus dem Produktstrom möglichst vollständig abgetrennt und zurückgewonnen werden. Da die Rh-Konzentration in typischen Hydroformylierungsreaktionen lediglich 20 bis 100 ppm beträgt und eine typische "world scale" Oxo-Anlage eine Jahresleistung von 200 000 Tonnen erzielt, müssen Trennapparate eingesetzt werden, die einerseits einen großen Durchsatz erlauben und andererseits das nur in geringen Mengen enthaltene Rh sicher abscheiden. Erschwerend kommt hinzu, dass die verwendeten Katalysator-Komplexe empfindlich auf Parameteränderungen reagieren (z.B. Temperaturänderung und/oder Änderung des CO-Partialdruckes in der Reaktionsmischung). Tritt eine solche Änderung auf kann es zu einer Desaktivierung des Katalysator-Komplexes kommen, die im schlimmsten Fall irreversibel ist. Auch die Phosphor-organischen Verbindungen die als Liganden Teil der Katalysatorkomplexe sein können, sind nicht unbegrenzt stabil, so können sie beispielsweise durch Feuchtigkeit, Sauerstoffeintrag oder zu hohe Temperaturen zersetzt werden, was ebenfalls eine Desaktivierung des Katalysators zur Folge hat. Ein desaktivierter Katalysator ist bestenfalls nur aufwändig zu reaktivieren. Die Katalysatorabtrennung muss deshalb besonders schonend erfolgen. Ein weiteres wichtiges Entwicklungsziel ist die Energie-Effizienz der Trennoperationen.

Die Membrantrenntechnik bietet sich zur Abtrennung von homogenen Katalysatorsystemen an, da sie weniger thermische Energie verbraucht und den Katalysator zu schonen vermag:

Über die Möglichkeiten des Einsatzes der Membrantechnologie zur Aufarbeitung von Hydroformylierungsgemischen berichten

Priske, M. et al.: Reaction integrated separation of homogeneous catalysts in the hydroformylation of higher olefins by means of organophilic nanofiltration. Journal of Membrane Science, Volume 360, Issues 1-2, 15 September 2010, Pages 77-83; doi:10.1016/j.memsci.2010.05.002.

Das Problem der Katalysatordesaktivierung während der Membranabtrennung wurde gelöst durch das in EP 1 931 472 B1 beschriebene Verfahren zur Aufarbeitung von Hydroformylierungsgemischen, bei dem sowohl im Feed, im Permeat und auch im Retentat der Membran ein bestimmter Kohlenmonoxid-Partialdruck aufrecht erhalten wird. Dadurch gelingt es erstmals, die Membrantechnologie wirksam in der industriellen Hydroformylierung einzusetzen. Eine mehrstufige Membranabtrennung ist hier nicht ersichtlich: So zeigt Figur 3 der EP 1 931 472 B1 die Katalysatorabtrennung mit Hilfe zweier hintereinander geschalteter Nanofiltrationen. Da zwischen den beiden Nanofiltrationen jedoch eine thermische Produktabtrennung mittels eines Dünnschichtverdampfers vorgesehen ist, bilden die beiden Nanofiltrationen keine Membrankaskade.

Ein weiteres membrangestütztes Verfahren zur Katalysatorabtrennung von homogen katalysierten Reaktionen wie insbesondere der Hydroformylierung ist aus der WO 2013/034690 A1 bekannt. Die dort gezeigte, einstufige Membrantechnik ist besonders auf die Bedürfnisse eines als der Reaktionszone genutzten Strahlschlaufenreaktors eingerichtet.

Eine membrangestützte Abscheidung von Homogenkatalysator aus Hydroformylierungsgemischen ist auch in der bisher unveröffentlichten deutschen Patentanmeldung DE 10 2012 223 572 A1 beschrieben. Zwar sind hier mehrere Nanofiltrationen innerhalb einer Anlage vorgesehen, allerdings sind die einzelnen Nanofiltrationen jeweils einem Reaktor einer Reaktorkaskade zugeordnet. Die Nanofiltrationen selbst bilden ebenfalls keine Membrankaskade.

Dasselbe gilt für die ebenfalls noch unveröffentlichte DE 10 2013 208 759 A1, welche sich unter anderem mit der Regelung der Trenntemperatur und des Rückhalts einer Membrantrenneinheit befasst.

Schließlich beschreibt die ebenfalls noch unveröffentlichte DE 10 2013 203 117 A1 den Einsatz einer Abtriebskaskade und einer Verstärkerkaskade zur Katalysatorabtrennung bzw. Hochsiederausschleusung aus einer homogen katalysierten Hydroformylierung. Einzelheiten zur Temperaturführung innerhalb der Membrankaskade werden nicht erörtert.

Schmidt et al. Beschreiben in der Zeitschrift Computer Aided Chemical Engineering Band 30, (2012), S. 727-731 ebenfalls die Verwendung von Membrankaskaden in der Hydroformylierung.

Die Verwendung von Membrankaskaden mit Temperaturkontrolle zur Trennung von Stoffgemischen in der Hydroformulierung sind in EP0823282 und EP0374615 offenbart.

Wenngleich im Stand der Technik schon Maßnahmen zur Abscheidung von homogenen Katalysatorsystemen aus Reaktionsmischungen beschrieben wurden besteht weiterhin das Problem der Verfügbarkeit von effektiven Materialien zu vertretbaren Preisen.

Zwar existieren Materialien, welche das homogen gelöste Katalysatorsystem zurückzuhalten vermögen, allerdings ist der Rückhalt vergleichsweise gering, sodass eine entsprechend große Membranfläche vorgesehen sein muss. Dies gilt umso mehr, wenn sehr große Mengen an Stoffgemisch aufzutrennen sind, etwa bei der Katalysatorabtrennung einer Oxoanlage im "World Scale"-Format. Die hierfür erforderlichen, riesenhaften Membranflächen erhöhen die Investitionskosten signifikant, so dass die Membran gestützte Katalysatorabscheidung gegenüber einer klassischen thermischen Abtrennung nicht immer wirtschaftlich ist.

Im Lichte dieses Standes der Technik liegt der Erfindung mithin die Aufgabe zugrunde ein membranbasiertes Verfahren zur Trennung von Stoffgemischen anzugeben, welches mit einer möglichst geringen Membranfläche auskommt und dennoch die geforderte Trennaufgabe und Trennleistung erfüllt.

Gelöst wird diese Aufgabe durch den Einsatz einer Membrankaskade mit sinkender Trenntemperatur.

Gegenstand der Erfindung ist mithin ein Verfahren zur Trennung eines Stoffgemisches mit Hilfe einer mindestens zwei Stufen aufweisenden Membrankaskade, bei welchem in jeder Stufe eine Stofftrennung an mindestens einer Membran bei einer der jeweiligen Stufe zugeordneten Trenntemperatur erfolgt, bei welchem die jeweilige Trenntemperatur - in Richtung des anströmenden Stoffgemisches gesehen - von Stufe zu Stufe sinkt.

Die Erfindung beruht auf der überraschenden Erkenntnis, dass sich die Fläche einer Membrankaskade - und damit ihr Bedarf an trennaktiven Membranmaterial -unter Beibehaltung der geforderten Trennleistung (Rückhalt und Volumenstrom des zu verarbeiteten Feeds) optimieren lässt, wenn die Trenntemperatur innerhalb der Kaskade von Stufe zu Stufe abgesenkt wird.

Diese Erkenntnis ist insoweit überraschend, als innerhalb von bekannten Membrankaskaden stets dieselbe Trenntemperatur beibehalten wurde:

Sofern man die üblichen Wärmeverluste außer Acht lässt, arbeitet eine herkömmliche Membrankaskade nämlich isotherm. Mit der Einstellung der Temperatur des Feeds der Membrankaskade wurde mithin die Trenntemperatur für alle Stufen identisch vorgegeben.

Dem gegenüber lehrt die Erfindung, die Trenntemperatur innerhalb der Kaskade für jede einzelne Stufe gezielt und gesondert einzustellen und zwar so, dass die Trenntemperatur stromabwärts von Stufe zu Stufe sinkt.

Die Erfindung macht sich die Beobachtung zu Nutze, dass der Rückhalt der einzelnen Membrantrennstufe abhängig ist von der Trenntemperatur dieser Stufe: Grundsätzlich gilt nämlich, dass mit sinkender Temperatur der Rückhalt der Membran steigt. Mit steigendem Rückhalt wird auch der Permeatfluss geringer, was wiederum eine kleinere Membranfläche innerhalb dieser Stufe ermöglicht.

Dies bedeutet, dass die Gesamtmembranfläche einer Kaskade sich optimieren lässt, indem man den Rückhalt in der ersten Stufe eher klein einstellt ihn aber von Stufe zu Stufe entsprechend durch Absenkung der Temperatur steigert. Im Ergebnis bedeutet dies, dass die Membranen von Stufe zu Stufe dichter werden. Die damit abnehmenden Volumenströme des Permeats können genutzt werden die Membranflächen insgesamt abzusenken.

Allerdings bedeutet dies nicht, dass die Membranfläche von Stufe zu Stufe sinkt; im Gegenteil kann die Membranfläche zum Ende hin auch wieder zunehmen. Die genaue Größe der jeweiligen Membranfläche innerhalb der Stufe berechnet der Membrantechniker aufgrund der jeweiligen zu erfüllenden Trennaufgabe. Entsprechende Berechnungsmethoden werden im zitierten Lehrbuch von Melin / Rautenbach beschrieben.

Entscheidende Randbedingung bei der Optimierung der Gesamtmembranfläche ist die sinkende Trenntemperatur- Diese Erkenntnis gilt grundsätzlich für die Auftrennung jedweder Stoffgemische mithilfe einer Membrankaskade unabhängig von dem Stoffsystem und der Phasenzusammensetzung. Es muss lediglich eine für die Abtrennung der gewünschten Stoffkomponente geeignetes Membranmaterial eingesetzt werden. Der Begriff "Membranmaterial" bezeichnet den Werkstoff der trennaktiven Schicht einer Membran.

Zur Abtrennung von homogen gelösten Katalysatorsystemen aus Reaktionsgemischen werden vorzugsweise Membranen eingesetzt, die eine trennaktive Schicht aus einem Material, ausgewählt aus Celluloseacetat, Cellulosetriacetat, Cellulosenitrat, regenerierter Cellulose, Polyimiden, Polyamiden, Polyetheretherketonen, sulfonierten Polyetheretherketonen, aromatischen Polyamiden, Polyamidimiden, Polybenzimidazolen, Polybenzimidazolonen, Polyacrylnitril, Polyarylethersulfonen, Polyestern, Polycarbonaten, Polytetrafluorethylen, Polyvinylidenfluorid, Polypropylen, endständig oder seitenständig organomodifiziertem Siloxan, Polydimethylsiloxan, Siliconen, Polyphosphazenen, Polyphenylsulfiden, Polybenzimidazolen, Nylon-6,6, Polysulfonen, Polyanilinen, Polyurethanen, Acrylonitril/Glycidylmethacrylat (PANGMA), Polytrimethylsilylpropin, Polymethylpentin, Polyvinyltrimethylsilan, Polyphenylenoxid, Aluminiumoxiden mit verschiedenen Kristallstrukturen Titanoxiden, Siliciumoxiden, Zirkonoxiden, mit Silanen hydrophobisierte keramische Membranen, wie sie in EP 1 603 663 B1 beschrieben sind, Polymere mit intrinsischer Mikroporösität (PIM) wie PIM-1 und anderen, wie sie z. B. in EP 0 781 166 und in "Membranes" von I. Cabasso, Encyclopedia of Polymer Science and Technlogy, John Wiley and Sons, New York, 1987, beschrieben werden, aufweisen.

Die oben genannten Stoffe können insbesondere in der trennaktiven Schicht ggf. durch Zugabe von Hilfsstoffen vernetzt vorliegen oder als so genannte Mixed Matrix Membranen mit Füllstoffen wie z.B. Kohlenstoffnanoröhren, Metal Organic Frameworks oder Hollow Spheres sowie Partikel von anorganischen Oxiden oder anorganische Fasern, wie z. B. Keramik- oder Glasfasern versehen sein.

Besonders bevorzugt werden Membranen eingesetzt, die als trennaktive Schicht eine Polymerschicht aus endständig oder seitenständig organomodifiziertem Siloxan, Polydimethylsiloxan oder Polyimid aufweisen, die aus Polymeren mit intrinsischer Mikroporosität (PIM) wie PIM-1 aufgebaut sind, oder wobei die trennaktive Schicht über eine hydrophobierte keramische Membran aufgebaut ist. Eine detaillierte Beschreibung solcher Membranen zum Einsatz in der Hochsiederausschleusung findet sich in EP2401078A1.

Ganz besonders bevorzugt werden Membranen aus endständig oder seitenständig organomodifiziertem Siloxanen oder Polydimethylsiloxanen eingesetzt. Solche Membranen sind kommerziell erhältlich.

Neben den oben genannten Materialien können die Membranen weitere Materialien aufweisen. Insbesondere können die Membranen Stütz- oder Trägermaterialien aufweisen, auf die die trennaktive Schicht aufgebracht ist. Bei solchen Verbundmembranen liegt neben der eigentlichen Membrane noch ein Stützmaterial vor. Eine Auswahl von Stützmaterialien beschreibt EP0781166, auf welches explizit verwiesen wird.

Eine Auswahl von kommerziell erhältlicher Lösungsmittel stabiler Membranen sind die MPF und Selro Serien von Koch Membrane Systems, Inc., unterschiedliche Typen von Solsep BV, die Starmem™ Serie von Grace/UOP, die DuraMem™ und PuraMem™ Serie von Evonik Industries AG, die Nano-Pro Serie von AMS Technologies, die HITK-T1 von IKTS, sowie oNF-1, oNF-2 und NC-1 von der GMT Membrantechnik GmbH und die inopor® nano Typen der Inopor GmbH.

Neben dem Membranmaterial ist auch die Bauform der Membranmodule relevant für die Trennleistung der einzelnen Stufe: Bevorzugt wird die Membran als Spiralwickelelement ausgeführt. Alternativ können Membranen z.B. auch in Gestalt von Plattenmodulen, Kissenmodulen, Rohrmodulen, Schlauchmodulen, Kapillarmodulen, Hohlfasermodulen oder Membranscheiben eingesetzt werden.

Maßgebend für die benötigte Membranfläche zur Erfüllung der Trennaufgabe ist auch die Verschaltung der Stufen innerhalb der Kaskade:
Bei der Membrankaskade handelt es sich nämlich vorzugsweise um eine Verstärkungskaskade, also eine Verschaltungsanordnung, bei der die einzelnen Stufen in Richtung des Permeatflusses hintereinander geschaltet sind. Das erfindungsgemäße Konzept funktioniert bei einer Verstärkungskaskade besonders gut, da die Verschaltung in Richtung des sinkenden Permeatflusses erfolgt, was die Optimierung der gesamten Membranfläche besonders erfolgreich werden lässt.

Aus demselben Grunde wird die Verstärkungskaskade so ausgeführt, dass das resultierende Permeat sämtliche Stufen der Membrankaskade permeiert hat.

Um die den Rückhalt der Membrankaskade insgesamt zu verbessern, bietet es sich an, das Retentat zumindest einer Stufe zwecks eines weiteren Abtrennschritts zurückzuführen. Die Rückführung des Retentats erfolgt dementsprechend in den Feed derselben Stufe, aus der das Retentat rezykliert wurde und/oder in den Feed einer anderen, entgegen der Richtung des anströmenden Stoffgemisches gelegenen Stufe. Das rezyklierte Retentat wird stets mit einem Stoffstrom eines geringeren Aufarbeitungsgrades vermischt.

Denknotwendig weist eine Membrankaskade wenigstens zwei Stufen auf. Wirtschaftlich kann aber auch eine drei- oder vierstufige Membrankaskade sein.

Wie bereits oben erwähnt, ist eine Membrankaskade als isotherm aufzufassen, sofern keine weiteren technischen Maßnahmen zur Temperaturführung innerhalb der Membran vorgesehen sind. Die natürlicherweise eintretenden Wärmeverluste innerhalb der Kaskade in Richtung des Permeats werden in der Regel nicht ausreichen, um optimale Trenntemperaturen in den einzelnen Stufen zu erreichen. Aus diesem Grunde ist es angezeigt, jeder Stufe der Membrankaskade eine Temperiereinheit zuzuordnen, welche die Temperatur des Feeds der jeweiligen Stufe auf die jeweilige, der Stufe zugeordneten Trenntemperatur einstellt. Die Temperiereinheit muss nicht zwangsläufig innerhalb der Membrankaskade angeordnet sein, sondern kann sich auch außerhalb derselben befinden. So kann insbesondere das aufzutrennende Stoffgemisch bereits mit der für die erste Stufe erforderlichen Temperatur bereitgestellt werden.

Bei der Temperiereinheit handelt es sich einfachstenfalls um einen Kühler, da die Temperatur in Richtung des Permeatflusses jeweils abgesenkt wird. Die Anordnung eines Kühlers innerhalb einer Membrankaskade bedeutet selbstverständlich, dass die Membrankaskade einen thermischen Energiebedarf in Gestalt von kühlendem Kühlmittel hat. Die Betriebskosten einer erfindungsgemäßen Membrankaskade können daher höher sein als die einer herkömmlichen Membrankaskade, die lediglich mechanische Leistung aufnimmt. Allerdings können diese höheren Betriebskosten durch geringere Investitionskosten oder einen verbesserten Rückhalt und damit einer besseren Produktreinheit wieder ausgeglichen werden, sodass die erfindungsgemäße Membrankaskade trotz ihres Kühlmittelbedarfs wirtschaftlicher ist als herkömmliche Anlagen.

Das erfindungsgemäße Verfahren beinhaltet die Abtrennung von homogen gelösten Katalysatorsystemen, da es sich hier als besonders kosteneffizient erwiesen hat. Das Stoffgemisch stammt ursprünglich aus seiner homogen katalysierten chemischen Reaktion und umfasst zumindest ein Produkt der Reaktion, zumindest ein nicht umgesetztes Edukt der Reaktion, sowie das der Reaktion gegenwärtige Katalysatorsystem und/oder zumindest ein Bestandteil und/oder ein Abbauprodukt desselben, wobei das Katalysatorsystem bzw. dessen Bestandteil bzw. dessen Abbauprodukt in dem Stoffgemisch gelöst ist.

Bei der Reaktion handelt es sich um eine Hydroformylierung. Dementsprechend handelt es sich bei dem Produkt um einen Aldehyd oder um einen Alkohol, bei den Edukten um ein Olefin und um Synthesegas. Bei dem Katalysatorsystem handelt es sich vorzugsweise um einen metall-organischen Komplex des Rhodiums (, wobei aber auch Komplexe der anderen Übergangsmetalle aus den Gruppen 7-9 des Periodensystems der Elemente Verwendung finden können), der beispielsweise eine phosphor-organische Verbindung als Liganden enthalten kann. Auf diesem speziellen Einsatzgebiet konnte das erfindungsgemäße Verfahren besonders vorteilhaft eingesetzt werden.

Offenbart ist auch eine Membrankaskade, welche zum erfindungsgemäßen Trennen eines Stoffgemisches bestimmt ist. Die Membrankaskade ist insbesondere als Verstärkungskaskade ausgeführt, umfassend mindestens zwei hintereinander angeordnete Stufen, bei welcher jeder Stufe eine Temperiereinheit zugeordnet ist, mittels welcher die Temperatur des Feeds der jeweiligen Stufe auf eine jeweilige, der Stufe zugeordneten Trenntemperatur einstellbar ist, und bei welcher die jeweiligen Trenntemperaturen so eingestellt sind, dass - in Richtung des anströmenden Stoffgemisches gesehen - die jeweilige Trenntemperatur von Stufe zu Stufe sinkt.

Offenbart ist auch die Verwendung dieser Membrankaskade zur Abtrennung eines gelösten Katalysatorkomplexes und/oder zumindest eines Bestandteils und/oder eines Abbauproduktes desselben aus einem ursprünglich aus einer in Gegenwart des Katalysatorkomplexes homogen katalysierten chemischen Reaktion stammenden Stoffgemisches.

Da das erfindungsgemäße Verfahren bevorzugt zur Katalysatorabtrennung innerhalb der homogen katalysierten, industriellen Hydroformylierung eingesetzt wird, ist auch ein Verfahren zur Hydroformylierung von ethylenisch ungesättigten Verbindungen durch Umsetzung mit Kohlenmonoxid und Wasserstoff in Gegenwart eines Katalysatorsystems, welches eine gelöste Metallkomplexverbindung eines Metalls der achten Nebengruppe des Periodensystems der Elemente mit wenigstens einer Organophosphorverbindung als Liganden enthält, bei welchem ein Reaktionsgemisch erhalten wird, welches neben Produkten der Hydroformylierung, nicht umgesetzte Edukte und das Katalysatorsystem oder zumindest Bestandteile und/oder Abbauprodukte desselben gelöst enthält, wobei das Reaktionsgemisch einer Katalysatorabtrennung zugeführt wird, in welcher das Katalysatorsystem bzw. dessen Bestandteile und/oder Abbauprodukte zwecks Rückführung in die Hydroformylierung aus dem Reaktionsgemisch zumindest teilweise mittels Membrantechnologie abgetrennt wird, bei welchem die Katalysatorabtrennung eine mindestens zwei Stufen aufweisende Membrankaskade umfasst, bei welcher in jeder Stufe eine Stofftrennung an mindestens einer Membran bei einer der jeweiligen Stufe zugeordneten Trenntemperatur erfolgt, wobei - in Richtung des anströmenden Reaktionsgemisches gesehen - die jeweilige Trenntemperatur von Stufe zu Stufe sinkt offenbart.

Die Erfindung soll nun anhand von Ausführungsbeispielen näher erläutert werden. Hierfür zeigen:

Figur 4: zweistufige Membrankaskade (erfindungsgemäß);
Figur 5: dreistufige Membrankaskade (erfindungsgemäß);
Figur 6: vierstufige Membrankaskade (erfindungsgemäß);
Figur 7: Abhängigkeit der Permeabilität von der Temperatur;
Figur 8: Abhängigkeit des Rückhalts von der Temperatur.

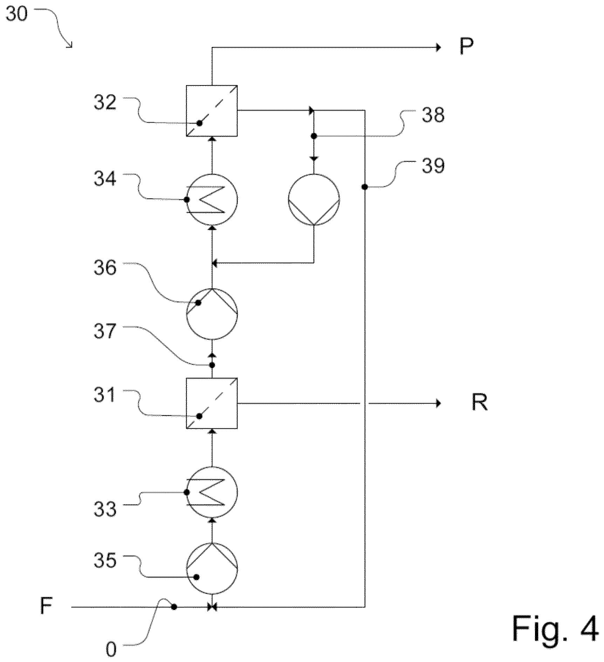

Figur 4 zeigt eine erste Ausführungsform der Erfindung in Gestalt einer zweistufigen Verstärkungskaskade, welche zur Durchführung eines erfindungsgemäßen Verfahrens geeignet ist. Bei dem Feed F der zweistufigen Verstärkerkaskade 30 handelt es sich um den Austrag einer homogen katalysierten Hydroformylierung. Genauer gesagt werden hier $C_5$-Olefine (Pentene) mit Wasserstoff und Kohlenmonoxid in Gegenwart eines Rhodium-Phosphit-Katalysatorsystems zu Hexanalen umgesetzt.

Beim Reaktionsaustrag handelt es sich um ein Gemisch aus beiden gebildeten Hexanalen (n-Hexanal und 2-Methylpentanal), in dem der homogen gelöste Katalysator in einer Konzentration von 37ppm (massenbezogen) vorliegt. Der Umsatz an Pentenen beträgt 99% und die Regioselektivität zum linearen Produkt beträgt 67%. Dies entspricht einer Reaktion bei einer Temperatur von 100°C, einem Druck von 40bar, einem CO/$H_2$-Verhältnis von 1, sowie einer Verweilzeit im Reaktor von 2 Stunden. Der Reaktionsaustrag umfassend die gebildeten Hexanale, nicht umgesetzte Pentene, gelöstes Synthesegas und das Katalysatorsystem werden als Feed F der Verstärkerkaskade 30 aufgegeben. Erforderlichenfalls kann zuvor eine Entgasung des Reaktionsaustrags erfolgen, wobei eine Restmenge an Synthesegas in dem Feed erhalten bleiben muss, um den Organophosphorliganden gegen Desaktivierung zu stabilisieren.

Die Verstärkerkaskade 30 weist zwei Stufen 31 und 32 auf. Beide Stufen 31, 32 werden jeweils von zwei in Serie geschalteten Membranmodulen gebildet, die jedoch wie eine einzelne Membran aufzufassen sind. Als Membranmaterial wird eine Silan-modifizierte Keramik basierend auf einem ZrO2 Träger und einer Porengröße von 3 nm eingesetzt, als Modulbauform wurden Rohrmodule vom Typ Inopor M07-19-41-L mit einer Länge von 40 Zoll und einer Fläche von 2.54m$^2$ gewählt. gewählt

Die Besonderheit der erfindungsgemäßen Verstärkerkaskade 30 besteht darin, dass im Feed jeder Membranstufe jeweils eine der jeweiligen Stufe 31, 32 zugeordnete Temperiereinrichtung 33, 34 vorgesehen ist. Bei den Temperiereinrichtungen 33, 34 handelt es sich um thermostatisch geregelte Kühler, welche den Feed der jeweiligen Stufen 31, 32 auf eine der jeweiligen Stufe zugeordneten Trenntemperatur einstellen. Die Trenntemperaturen der Stufen sind so gewählt, dass die Trenntemperatur in Richtung des anströmenden Feeds F gesehen sinkt:
So stellt die erste Temperiereinrichtung 33 die Trenntemperatur der ersten Stufe 31 auf 43,4 °C ein; währenddessen die Temperiereinrichtung 34 der zweiten Stufe 32 die Trenntemperatur der zweiten Stufe 32 auf 30 °C festlegt.

Als Transmembrandruck wird in jeder Stufe 60 bar eingestellt, wofür im Feed der jeweiligen Stufe jeweils eine Pumpe 35, 36 vorgesehen ist.

Die Pumpe der ersten Stufe 35 fördert den Feed F durch die erste Temperiereinrichtung 33, sodass diese auf die erste Stufe 31 gelangt. Dort findet eine Stofftrennung dergestalt statt, dass die in dem Reaktionsgemisch enthaltenen Produkte und Edukte die Membranen bevorzugt, das heißt schneller, überwinden und sich dementsprechend in dem Permeat 37 der ersten Stufe anreichern. Das Katalysatorsystem hingegen vermag die Membran nicht so schnell zu überwinden, so dass es sich im Retentat der ersten Stufe anreichert, welches als resultierendes Retentat R aus der Membrankaskade 30 abgezogen und zurück in den Hydroformylierungsreaktor geführt wird.

Keine Membran ist völlig dicht für das Katalysatorsystem. Deswegen enthält auch das Permeat 37 der ersten Stufe 31 Rhodium und Organosphosphor-Ligand oder dessen Abbauprodukte. Zur weiteren Abtrennung des Katalysatorkomplexes wird das Permeat 37 der ersten Stufe 31 als Feed der zweiten Stufe 32 aufgegeben. Zum Ausgleich des erlittenen Druckverlustes in Höhe des über die erste Stufe wirkenden Transmembrandruckes wird der Druck des Permeats 37 mit Hilfe der Pumpe 36 wieder erhöht. Sodann findet eine Abkühlung in der Temperiereinrichtung 34 statt, damit in der zweiten Stufe 32 wieder eine Membrantrennung auf demselben Druckniveau jedoch bei geringerer Trenntemperatur stattfinden kann.

Das Permeat der zweiten Stufe 32 wird als resultierendes Permeat P aus der zweistufigen Verstärkungskaskade 30 ausgeschleust. Es ist weitestgehend frei von dem Katalysatorsystem oder dessen etwaiger Abbauprodukte. Es kann nun einer destillativen Produktabtrennung zugeführt werden, in welcher das eigentliche Zielprodukt (Hexanal) von in Nebenreaktionen gebildeten Hochsiedern getrennt werden kann.

Das Retentat 38 der zweiten Stufe 32 wird indes innerhalb der Membrankaskade 30 rezykliert und zwar einmal über eine Rückführung innerhalb der zweiten Stufe 32, wofür eine Rückführpumpe innerhalb der Rückführung 38 angeordnet ist, sowie über eine zweite Rückführung 39, welche das Retentat der zweiten Stufe 32 in dem Feed der ersten Stufe 31 zurückführt. Die zweite Rückführung 39 kommt ohne eine eigene Pumpe aus, da die Pumpe 35 vor der ersten Stufe das rezyklierte Retentat der zweiten Stufe 32 mit ansaugt.

Der Wert $RR_{int}$ wird in der zweiten Stufe auf 12,1 kmol/kmol eingestellt und in der ersten auf 10,5 kmol/kmol. Dies bedeutet, dass (molmengenbezogen) 12,1 bzw. 10,5 mal mehr intern rezirkuliert wird (z.B. Strom 38), als extern in die darunterliegende Stufe (z.B. Strom 39, oder in der ersten Stufe an das globale Retentat) gefördert wird.

Aufgrund der sinkenden Trenntemperatur von 43,4 °C in der ersten Stufe auf 30 °C in der zweiten Stufe ist der Rückhalt der ersten Stufe geringer als der der zweiten Stufe. In der ersten Stufe beträgt der Rückhalt 88.7%, während der Rückhalt in der zweiten Stufe 91.3% beträgt.

Wegen des geringeren Rückhalts ist der Permeatfluss der ersten Stufe 31 (also der Volumenstrom des Permeats 37) deutlich größer als der Permeatfluss der zweiten Stufe 32 (also der Volumenstrom des resultierenden Permeats P). Dementsprechend ist auch die Membranfläche der ersten Stufe mit 163 m² deutlich größer als die der zweiten Stufe mit 81,4 m2.

Insgesamt benötigt die in Figur 4 dargestellte zweistufige Membrankaskade 30 eine Gesamtmembranfläche von 244,4 m². Damit wird ein Gesamtrückhalt bezogen auf den Katalysatorkomplex von 98,33 % erzielt. Dies bedeutet, dass 98,33 % des mit dem Feed eingebrachten Katalysators wiedergewonnen werden kann und die Membrankaskade 30 wieder übers resultierende Retentat R in Richtung des Reaktors verlässt. Die übrigen 1,67 % des Katalysatorsystems gehen mit dem Permeat P verloren.

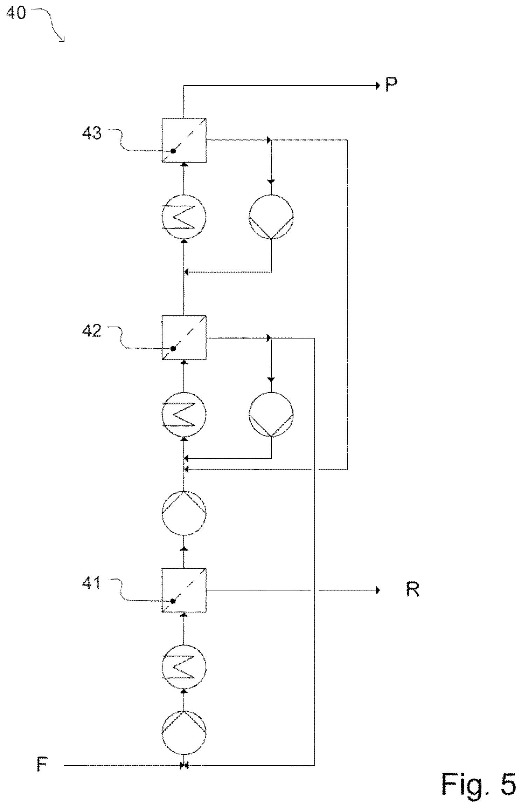

Um Katalysatorverluste über das resultierende Permeat P zu reduzieren muss der Katalysatorrückhalt weiter gesteigert werden. Hierfür bietet es sich eine dreistufige Verstärkerkaskade an, wie sie beispielsweise in Figur 5 dargestellt ist.

Wie ihr Name schon sagt umfasst die dreistufige Verstärkerkaskade 40 drei Stufen 41, 42, 43, die analog zu der in Figur 4 gezeigten, zweistufigen Verstärkerkaskade 30 miteinander verschaltet sind. Das resultierende Retentat R der Kaskade 40 wird von der ersten Stufe 41 abgezogen. Das resultierende Permeat P permeiert sämtliche Stufen 41, 42 und 43. Die Retentate der zweiten und dritten Stufe werden rezykliert und zwar jeweils teilweise in den Feed derselben Stufe und in den Feed der vorhergehenden Stufe. Der Wert $RR_{int}$ beträgt in der ersten Stufe 0,5 kmol/kmol, in der zweiten Stufe 5,1 kmol/kmol und in der dritten Stufe 16,6 kmol/kmol.

Das verwendete Membranmaterial ist einheitlich und dasselbe wie bei der in Figur 4 gezeigten zweistufigen Verstärkerkaskade 30. Es wurde auch der identische Transmembrandruck von 60 bar gewählt. Die Trenntemperatur nimmt wieder in Richtung des Permeatflusses ab von 88,9 °C in der ersten Stufe 41, über 73,1 °C in der zweiten Stufe 42, bis auf 30,17 °C in der dritten Stufe 43 ab. Als Membranfläche wurde in der ersten Stufe 64,5 m² gewählt; bereitgestellt durch zwei in Serie geschaltete "Inopor M07-19-41-L"-Module mit einer Länge von 40 Zoll und einer Fläche von jeweils 2.54m² gewählt. Die zweite Stufe 42 weist eine Membranfläche von 61 m² auf, ebenfalls realisiert über zwei in Serie geschaltete Membranmodule. In der dritten und letzten Stufe 43 wurde die Membranfläche allerdings auf 81,2 m² angehoben, bereitgestellt von einer einzigen Membran. Insgesamt benötigt die dreistufige Verstärkungskaskade 206,7 m² kostspieliges Membranmaterial. Ihr auf den Katalysatorkomplex bezogener Rückhalt beträgt jedoch 99,24 %. Im Vergleich mit der zweistufigen Verstärkerkaskade 30 erzielt die dreistufige Verstärkerkaskade mithin einen besseren Rückhalt mit einer geringeren Membranfläche. Die in Figur 5 dargestellte dreistufige Ausführungsform ist somit deutlich effektiver als die in Figur 4 gezeigte zweistufige Membrankaskade.

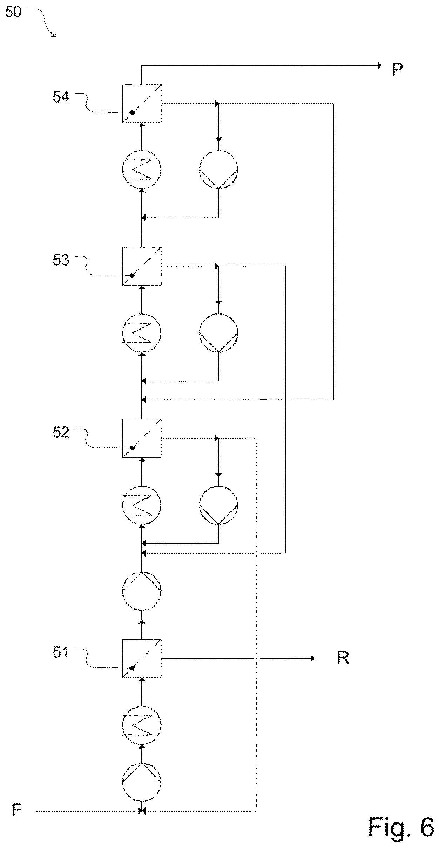

Um den Rückhalt der Membrankaskade noch weiter zu steigern, kann ein vierstufiges System vorgesehen werden. Figur 6 zeigt eine entsprechende vierstufige Verstärkerkaskade 50. Ihre Verschaltung ist analog zu der dreistufigen Verstärkerkaskade 40 und zweistufigen Verstärkerkaskade 30. Die Zusammensetzung des Feeds F und das Membranmaterial und die Modulbauform entsprechenden Membrankaskaden aus den Figuren 4 und 5. Die Stufen 51, 52, 53 und 54 wurden wie folgt gewählt:

Erste Stufe 51:    Trenntemperatur 90 °C, Transmembrandruck 60 bar, Anzahl der Membranen 2, Membranfläche 53,2 m2, $RR_{int}$ 0,3 kmol/kmol.

Zweite Stufe 52:    Trenntemperatur 85,5 °C, Transmembrandruck 60 bar, Anzahl der Membranen 2, Membranfläche 73,9 m2, $RR_{int}$ 7,8 kmol/kmol.

Dritte Stufe 53:    Trenntemperatur 80,9 °C, Transmembrandruck 60 bar, Anzahl der Membranen 2, Membranfläche 66,5 m2, $RR_{int}$ 4,8 kmol/kmol.

(fortgesetzt)

Vierte Stufe 54: Trenntemperatur 63,9 °C, Transmembrandruck 60 bar, Anzahl der Membranen 2 mit einer Gesamtfläche von 53,3 m2, $RR_{int}$ 6,1 kmol/kmol.

Die vierstufige Membrankaskade 50 erreicht einen Gesamtrückhalt von 99,5 % bei einer Gesamtmembranfläche von 245,9 m². Die Membranfläche ist somit etwa genau so groß wie die der zweistufigen Verstärkerkaskade 30 aus Figur 4, der Rückhalt ist jedoch deutlich besser.

Zum Vergleich wurden die drei erfindungsgemäßen Verstärkerkaskaden 30, 40 und 50 unverändert betrieben, jedoch bei einer einheitlichen Trenntemperatur innerhalb der Kaskade. Die Ergebnisse sind in Tabelle 1 dargestellt.

| Stufenzahl | Eingestellte isotherme Temperatur [°C] | Äquivalente Gesamtmembranfläche [m²] | Katalysatorrückhalt bei isothermer Temperaturführung [%] | Vergleichswert Katalysatorrückhalt bei nichtisothermer Führung [%] |
|---|---|---|---|---|
| 2 | 38.64 | 244.4 | 97.91 | 98.33 |
| 3 | 73.88 | 206.7 | 98.08 | 99.24 |
| 4 | 80.44 | 245.9 | 99.41 | 99.50 |

Der Vergleich zeigt, dass die erfindungsgemäßen Membrankaskaden 30, 40 und 50 bei identischer Membranfläche einen höheren Rückhalt bieten als die entgegen der erfindungsgemäßen Lehre isotherm betriebenen Kaskaden.

Dies bedeutet umgekehrt, dass derselbe Rückhalt mit einer geringeren Membranfläche erzielt werden kann.

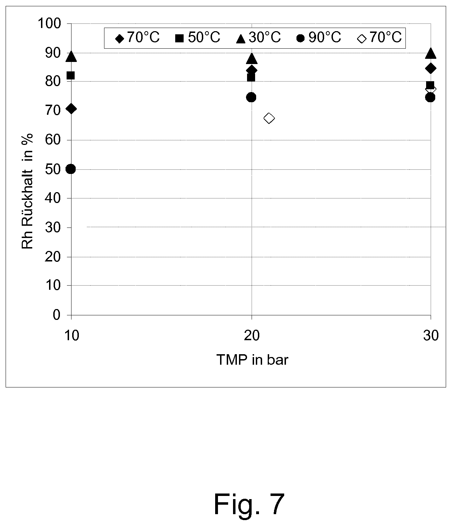

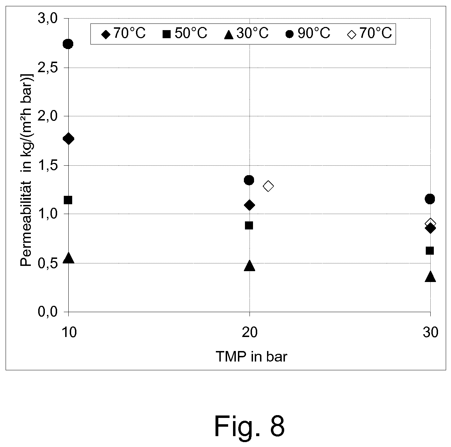

Die Figuren 7 und 8 zeigen den erfindungsgemäß genutzten Effekt der Temperaturabhängigkeit des Rückhalts bzw. der Permeabilität. Die Figuren 7 und 8 zeigen die Ergebnisse bzgl. Katalysatorrückhalt (Rh Rückhaltung) und Permeabilität. Je niedriger die Temperatur ist, desto höher ist der Rückhalt und so geringer ist die Permeabilität.

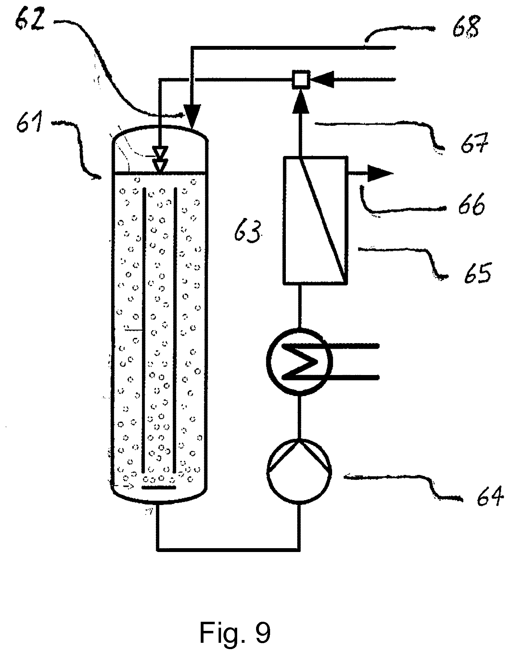

In einer, wie in Fig. 9 dargestellten, Versuchsanlage mit einem Strahlschlaufenreaktor (61) wurden Hydroformylierungsreaktionen von 1-Penten (68) mit Synthesegas (62) zu den entsprechenden Aldehydisomeren durchgeführt. Im von einer Peripheralradpumpe (64) getriebenen Flüssigkeitsumlauf (63) erfolgte eine Abtrennung und Rückführung des Katalysator-Ligandsystems mittels einer Membrantrennstufe (65) zur kontinuierlichen Wiedereinsetzung des Katalysator-Ligandsystems in der Hydroformylierungsreaktion im Strahlschlaufenreaktor (61).

Für die Reaktion wurden dem Reaktor 1-Penten (68) unter Sauerstoffausschluss kontinuierlich entsprechend der Reaktionsproduktabfuhr über das Permeat der Membrantrennstufe zugeführt. Der Katalysatorpräkursor war Rhodiumacetylacetonatodicarbonyl (CAS-Nr. 14847-82-9). Als Ligand wurde Alkanox P-24 (CAS-Nr. 26741-53-7) eingesetzt. Die Rhodiumkonzentration und die Ligandkonzentration im Schlaufenreaktor wurde durch eine kontinuierliche Nachdosierung konstant auf 10 mg/kg bzw. 1170 mg/kg gehalten. Die Reaktion wurde unter 50 bar Synthesegasdruck ($CO/H_2$, Massenverhältnis 1:1), bei 110 °C durchgeführt.

Das Reaktionsprodukt wurde kontinuierlich über eine als einstufige Nanofiltrationsmembran ausgeführte Membrantrennstufe (65) geführt. Der benötigte transmembrane Druck wird durch den Reaktordruck und einem geregelten permeatseitigen Druck aufbaut. Die gewünschte Überströmung von 500 kg/h über die Hochdruckseite der Membran wird über die Peripheralradpumpe (69) eingestellt.

Im Membranmodul der Membrantrennstufe (65) war ein Prototyp einer durch Silanisierung hydrophobierten Membran als Monokanalrohr vom Fraunhofer-Institut für Keramische Technologien und Systeme IKTS verbaut. Der Träger bestand aus $Al_2O_3$ mit einer mittleren Porengröße von 3 $\mu$m und einer hydrophobierten Membranschicht basierend auf einer $ZrO_2$ Schicht mit einem mittleren Porendurchmesser von 3 nm.

Bei einer Kanallänge von 500mm und einem Innendurchmesser von 7 mm ergibt sich eine aktive Membranfläche von ca. 100 cm². Die Membran wurde mit 4,4 m/s überströmt. Es wurden Temperaturen an der Membran von 30 bis 90°C. Zur Stabilisierung des Katalysator-Ligandkomplexes wurde auf der Permeatseite ein Synthesegasdruck ($CO/H_2$, Massenverhältnis 1:1) von 10 bar gehalten, wodurch bei einem retentatseitigen Druck von 20 bis 40bar ein transmembranen Druck von 10 bis 30 bar eingestellt wurde.

In der Membrantrennstufe wurde dem System über die Membran Permeat (66), das vorwiegend aus Reaktionsprodukt besteht, entnommen. Der Katalysator sowie der Ligand Alkanox wurde von der Membran weitestgehend zurückgehalten und reichert sich im Retentat (67) an. Das Retentat (67) wurde kontinuierlich zurück in den Strahlschlaufenreaktor (61) geführt.

Die Prozesskette wurde an Hand von Mess- und Analysendaten, die durch gaschromatographische Analyse, HPLC-Analyse, Atomabsorptionsspektroskopie und Optische Emissionsspektrometrie mit induktiv gekoppeltem Hochfrequenzplasma erhalten wurden, ausgewertet. Die Reaktion wurde hinsichtlich des Umsatzes an 1-Penten sowie der Ausbeute und der Selektivität an Aldehyd untersucht. Die Membrantrennstufe (65) wurde hinsichtlich Permeatfluss und Rückhalt für Rhodium untersucht. Die Zusammensetzung des Reaktionsaustrages war wie folgt:

| 1-Penten | 2-M.pentanal | Hexanal | Rest |
|----------|--------------|---------|------|
| 3,7 % | 53,3 % | 42,2 % | 0,8 % |

**Bezugszeichenliste**

| | |
|---|---|
| 0 | Stoffgemisch |
| 1 | Membran |
| F | Feed |
| P | (resultierendes) Permeat |
| R | (resultierendes) Retentat |
| 10 | Verstärkerkaskade (Stand der Technik) |
| 11 | erste Stufe der Verstärkerkaskade |
| 12 | zweite Stufe der Verstärkerkaskade |
| 13 | Förderpumpe der ersten Stufe |
| 14 | Retentat der ersten Stufe |
| 15 | Permeat der ersten Stufe |
| 16 | Hochdruckpumpe |
| 17 | Retentat der zweiten Stufe |
| 18 | Permeat der zweiten Stufe |
| 20 | Abtriebskaskade (Stand der Technik) |
| 21 | erste Stufe der Abtriebskaskade |
| 22 | zweite Stufe der Abtriebskaskade |
| 23 | Druckerhöhungspumpe |
| 24 | Retentat der ersten Stufe |
| 25 | Permeat der ersten Stufe |
| 26 | Permeat der zweiten Stufe |
| 27 | Retentat der zweiten Stufe |
| 30 | zweistufige Verstärkerkaskade |
| 31 | erste Stufe der Verstärkerkaskade |
| 32 | zweite Stufe der Verstärkerkaskade |
| 33 | erste Temperiereinrichtung |
| 34 | zweite Temperiereinrichtung |
| 35 | Pumpe der ersten Stufe |
| 36 | Pumpe der zweiten Stufe |
| 37 | Permeat der ersten Stufe |
| 38 | Rückführung vor die zweite Stufe |
| 39 | Rückführung vor die erste Stufe |
| 40 | dreistufige Verstärkerkaskade |
| 41 | erste Stufe der Verstärkerkaskade |
| 42 | zweite Stufe der Verstärkerkaskade |
| 43 | dritte Stufe der Verstärkerkaskade |
| 50 | vierstufige Verstärkerkaskade |
| 51 | erste Stufe der Verstärkerkaskade |
| 52 | zweite Stufe der Verstärkerkaskade |
| 53 | dritte Stufe der Verstärkerkaskade |
| 54 | vierte Stufe der Verstärkerkaskade |
| 61 | Strahlschlaufenreaktor |
| 62 | Synthesegas |
| 63 | Flüssigkeitsumlauf |
| 64 | Peripheralradpumpe |

65    Membrantrennstufe
66    Permeat
67    Retentat
68    1-Penten

**Patentansprüche**

1. Verfahren zur Trennung eines Stoffgemisches mit Hilfe einer mindestens zwei Stufen aufweisenden Membrankaskade, bei welchem in jeder Stufe eine Stofftrennung an mindestens einer Membran bei einer der jeweiligen Stufe zugeordneten Trenntemperatur erfolgt, wobei das Stoffgemisch ursprünglich aus einer homogen katalysierten chemischen Reaktion stammt, und dass es zumindest ein Produkt der Reaktion, zumindest ein nicht umgesetztes Edukt der Reaktion, sowie das in der Reaktion gegenwärtige Katalysatorsystem und/oder zumindest ein Bestandteil und/oder ein Abbauprodukt desselben umfasst, wobei das Katalysatorsystem bzw. dessen Bestandteil bzw. dessen Abbauprodukt in dem Stoffgemisch gelöst ist, wobei es sich bei der Reaktion um eine Hydroformylierung handelt, wobei es sich bei dem Produkt um einen Aldehyd oder um einen Alkohol handelt, wobei es sich bei dem Edukt um ein Olefin oder um Synthesegas handelt und wobei es sich bei dem Katalysatorsystem um mit einem Organometallkomplex, insbesondere um mit Organophosphorkomplex komplexiertes Rhodium handelt,

   wobei jeder Stufe der Membrankaskade eine Temperiereinheit zugeordnet ist, welche die Temperatur des Feeds der jeweiligen Stufe auf die jeweilige, der Stufe zugeordneten Trenntemperatur einstellt,
   **dadurch gekennzeichnet,**
   **dass**, in Richtung des anströmenden Stoffgemisches gesehen, die jeweilige Trenntemperatur von Stufe zu Stufe sinkt.

2. Verfahren nach Anspruch 1,
   **dadurch gekennzeichnet,**
   **dass** es sich bei der Membrankaskade um eine Verstärkungskaskade handelt, bei der die einzelnen Stufen in Richtung des Permeatflusses hintereinander geschaltet sind.

3. Verfahren nach Anspruch 2,
   **dadurch gekennzeichnet,**
   **dass** das resultierende Permeat der Membrankaskade sämtliche Stufen der Membrankaskade permeiert hat.

4. Verfahren nach Anspruch 2 oder 3,
   **dadurch gekennzeichnet,**
   **dass** zumindest das Retentat einer Stufe in den Feed derselben Stufe und/oder in den Feed einer anderen, entgegen der Richtung des anströmenden Stoffgemisches gelegenen Stufe zurückgeführt wird.

5. Verfahren nach einem der Ansprüche 1 bis 4,
   **dadurch gekennzeichnet,**
   **dass** die Membrankaskade genau zwei oder genau drei oder genau vier Stufen aufweist.

6. Verfahren nach einem der Ansprüche 1 bis 5,
   **dadurch gekennzeichnet,**
   **dass** es sich bei zumindest einer Temperiereinheit um einen Kühler handelt.

**Claims**

1. Process for separating a composition of matter with the aid of a membrane cascade having at least two stages, in which a separation of matter is effected in each stage at at least one membrane at a separation temperature set for the particular stage, where the composition of matter originally originates from a homogeneously catalysed chemical reaction, and that it comprises at least one product of the reaction, at least one reactant unconverted in the reaction, and the catalyst system present in the reaction and/or at least one constituent and/or a degradation product thereof, where the catalyst system or the constituent thereof or the degradation product thereof is dissolved in the composition of matter, where the reaction is a hydroformylation, where the product is an aldehyde or an alcohol, where the reactant is an olefin or synthesis gas, and where the catalyst system is rhodium complexed with an organometallic complex, especially rhodium complexed with an organophosphorus complex,

where each stage of the membrane cascade has a dedicated temperature control unit which sets the temperature of the feed to the particular stage to the particular separation temperature set for the stage,
**characterized**
**in that** the particular separation temperature falls from stage to stage in the direction of the incoming composition of matter.

**2.** Process according to Claim 1,
**characterized**
**in that** the membrane cascade is an enriching cascade, where the individual stages are connected in series in the direction of the permeate flow.

**3.** Process according to Claim 2,
**characterized**
**in that** the permeate that results from the membrane cascade has permeated through all the stages of the membrane cascade.

**4.** Process according to Claim 2 or 3,
**characterized**
**in that** at least the retentate of one stage is recycled into the feed to the same stage and/or into the feed to another stage positioned counter to the direction of the incoming composition of matter.

**5.** Process according to any of Claims 1 to 4,
**characterized**
**in that** the membrane cascade has exactly two or exactly three or exactly four stages.

**6.** Process according to any of Claims 1 to 5,
**characterized**
**in that** at least one temperature control unit is a cooler.

**Revendications**

**1.** Procédé pour le fractionnement d'un mélange de substances à l'aide d'une cascade de membranes comportant au moins deux étages, dans lequel une séparation de substances s'effectue dans chaque étage sur au moins une membrane à une température de séparation affectée à l'étage respectif, le mélange de substances provenant initialement d'une réaction chimique à catalyse homogène, et comprenant au moins un produit de la réaction, au moins un produit de départ n'ayant pas réagi dans la réaction, ainsi que le système catalytique présent dans la réaction et/ou au moins un constituant et/ou un produit de dégradation de celui-ci, le système catalytique ou constituant de celui-ci ou produit de dégradation de celui-ci étant dissous dans le mélange de substances, la réaction consistant en une hydroformylation, le produit consistant en un aldéhyde ou en un alcool, le produit de départ consistant en une oléfine ou en gaz de synthèse et le système catalytique consistant en un complexe organométallique, en particulier en rhodium complexé avec un complexe organophosphoré,

à chaque étage de la cascade de membranes étant affectée une unité de régulation de la température, qui ajuste à la température de séparation respective, affectée à l'étage, la température de la charge de l'étage respectif,
**caractérisé**
**en ce que**, vu dans le sens d'écoulement du mélange de substances, la température de séparation respective décroît d'un étage à l'autre.

**2.** Procédé selon la revendication 1,
**caractérisé**
**en ce que** pour ce qui est de la cascade de membranes il s'agit d'une cascade d'enrichissement, dans laquelle les étages individuels sont montés les uns derrière les autres dans le sens du courant de perméat.

**3.** Procédé selon la revendication 2,

**caractérisé**

**en ce que** le perméat résultant de la cascade de membranes est passé à travers tous les étages de la cascade de membranes.

4. Procédé selon la revendication 2 ou 3,
**caractérisé**
**en ce qu'**au moins le rétentat d'un étage est renvoyé dans la charge du même étage et/ou dans la charge d'un autre étage situé à l'opposé du sens d'écoulement du mélange de substances.

5. Procédé selon l'une quelconque des revendications 1 à 4,
**caractérisé**
**en ce que** la cascade de membranes comporte exactement deux ou exactement trois ou exactement quatre étages.

6. Procédé selon l'une quelconque des revendications 1 à 5,
**caractérisé**
**en ce que** pour ce qui est de l'unité de régulation de la température il s'agit d'un refroidisseur.

## Fig. 1
(Stand der Technik)

Fig. 2

(Stand der Technik)

Fig. 3
(Stand der Technik)

Fig. 4

Fig. 5

Fig. 6

Fig. 7

Fig. 8

EP 3 027 298 B1

Fig. 9

25

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1931472 B1
- WO 2013034690 A1
- DE 102012223572 A1
- DE 102013208759 A1
- DE 102013203117 A1
- EP 0823282 A
- EP 0374615 A
- EP 1603663 B1
- EP 0781166 A
- EP 2401078 A1


**In der Beschreibung aufgeführte Nicht-Patentliteratur**

- Anlagenauslegung. Springer, 2004
- **FALBE, JÜRGEN.** New Syntheses with Carbon Monoxide. Springer, 1980
- **PRUETT, ROY L.** *Hydroformylation. Advances in Organometallic Chemistry.,* 1979, vol. 17, 1-60
- Hydroformylation (Oxo Synthesis, Roelen Reaction). **FROHNING, CARL D. ; KOHLPAINTNER, CHRISTIAN W.** Applied homogeneous catalysis with organometallic compounds. Wiley, 1996, 29-104
- Rhodium Catalyzed Hydroformylation. Catalysis by Metal Complexes. 2000, vol. 22
- **R. FRANKE ; D. SELENT ; A. BÖRNER.** Applied Hydroformylation. *Chem. Rev.,* 2012
- **PRISKE, M. et al.** Reaction integrated separation of homogeneous catalysts in the hydroformylation of higher olefins by means of organophilic nanofiltration. *Journal of Membrane Science,* 15. September 2010, vol. 360 (1-2), 77-83
- **SCHMIDT et al.** *Beschreiben in der Zeitschrift Computer Aided Chemical Engineering Band,* 2012, vol. 30, 727-731
- **MEMBRANES" VON I ; CABASSO.** Encyclopedia of Polymer Science and Technlogy. John Wiley and Sons, 1987
- *CHEMICAL ABSTRACTS,* 14847-82-9
- *CHEMICAL ABSTRACTS,* 26741-53-7